# EUROPEAN PATENT APPLICATION

(11) **EP 4 122 511 A1**
(43) Date of publication of application: **25.01.2023**
(21) Application number: 21186842.7
(22) Date of filing: 21.07.2021
(51) Int. Cl.: A61M 5/172, A61M 5/48, A61M 5/142, A61M 5/145, A61M 5/168, A61M 5/315

(54) **MONITORING A DISPENSING PROCESS WITH A DRUG DELIVERY DEVICE**

(71) Applicant: Ypsomed AG, 3401 Burgdorf (CH)
(72) Inventor: Brügger, Martin, 3065 Bolligen (CH); Bosshard, Simon Martin, 3006 Bern (CH); Schrul, Christian, 3400 Burgdorf (CH); Scheurer, Simon, 3006 Bern (CH)
(74) Representative: Meier Obertüfer, Jürg

(57) **Abstract**

The present invention relates to a method for monitoring a dispensing process of a liquid drug with a drug delivery device (1), the method comprising the steps of gradually increasing a motor drive current for a motor (33) of the drug delivery device (1); measuring, by a sensor, a position or movement of an output member (32) movable by the motor (33); determining a minimal motor drive current required to initially move the output member (32) from standstill and concluding, from an evolution of successively determined minimal motor drive current values, a delivery device operation status.

## Description

### FIELD OF THE INVENTION

The present invention relates to injection devices or medicament delivery devices for injecting, delivering, administering, infusing or dispensing substances and/or liquids such as insulin, hormone preparations or vaccines. It departs from a method for monitoring a dispensing process with the drug delivery device.

### BACKGROUND OF THE INVENTION

A variety of diseases exist that require regular treatment by subcutaneous administration of a medicament, and a number of drug delivery devices have been developed to support a patient in accurately and controllably delivering an amount of drug in a self-administration process. Delivery devices include injection devices that are removed from the injection site after each medication event or drug delivery process, as well as infusion devices with a cannula or needle that remains in the skin of the patient for a prolonged period of time.

By way of example, diabetes may be treated by self-administration of insulin or its derivatives with the help of multi-variable-dose insulin injection pens. An injection pen device generally has an elongate device body defining a longitudinal main device axis. An automatic injection device has a motor or a drive spring for biasing a plunger rod and shifting a piston in a container barrel, wherein the drive spring may have to be charged or strained manually prior to injection of a dose. A manually powered delivery drive requires a user to manually provide the energy to move the piston, for instance by applying a distal force component to the injection device.

The medicament dose to be injected may typically be manually selected by turning a dosage knob and observing the actual dialed dose from a dose window or display of the insulin pen. A dose is dispensed by inserting the needle into a suited portion of human skin and by moving the piston manually or by pressing a release button of an automatic injection device. Automatic injection devices include an electric motor or a biased drive element for automatically dispense the set dose.

Drug delivery device based therapies generally benefit from an electronic unit or control unit embedded or integrated in the delivery device, or being part of an auxiliary or supplemental electronic module or add-on device detachably attached to the delivery device. The electronic unit may monitor a drug delivery process, in order to proactively prevent false handling of the device and/or to keep track of the doses already applied, and generates data related to an instantaneous condition and/or use of the delivery device. Suitable sensors of the electronic unit readily detect a status or signal from any kind of indicating component of the delivery device, including user interface elements and actuators. A wireless communication unit of the electronic unit is provided to wirelessly communicate, specifically upload, drug information to a nearby mobile device, a dedicated medical gateway or a cloud server. The drug information includes at least a time stamp and the expelled dose, indicative of a time of a medication event and of a quantity of delivered drug. The drug information may be transmitted instantaneously, or stored in a memory unit connected to the processing unit, for later upload or batch transfer.

Delivery devices may include a safety mechanism adapted to recognize a malfunction, defects or errors inside the delivery device, in particular a malfunction with respect to the fluid path of the liquid drug.

For example, EP3213785 B1 discloses a drug pump including an electric motor for dispensing the drug. An electronic control circuit measures the pump motor current. If there is an occlusion in the system, an increase in reservoir fluid pressure will likely result. This may cause greater motor torque and current as the motor attempts to advance the reservoir plunger against this fluid pressure. If the measured motor current is some amount greater than a known, average baseline motor current, which may be established when there was no occlusion condition, then it is determined that an occlusion condition is likely to have occurred. If there was a failure of the gearbox causing the motor to be unable to rotate, the measured current would be high and an encoder would not increment. This is an indication of a drive system fault.

EP 3765125 A1 discloses a drug delivery device with an electric motor for moving a piston rod. The motor current is measured to energize the electromotor and used as a signal for the load delivered by the motor. The current signal is processed by a control module for detecting a blocking of the piston rod advancement.

WO 2011/044706 A1 suggests to use a force sensor to detect an increase in the motor torque and thus in the drive system in case of a malfunction.

Prior art approaches often suffer from a considerable time lag until a system error or a malfunction is detected as it takes some time until the current increases to a maximum value or reaches a predefined threshold. To avoid the time lag often additional force sensors or torque sensors are required. However, they need additional space and increase the production costs of the drug delivery device.

### DESCRIPTION OF THE INVENTION

It is an objective of the invention to provide a reliable monitoring of a dispensing process with a drug delivery device and to provide an early detection of a malfunction.

This objective is achieved by the method and the drug delivery device according to the independent claims. Preferred embodiments are evident from the dependent claims.

The method according to the invention concerns a monitoring of a dispensing process of a liquid drug from a reservoir or cartridge in a drug delivery device. The drug delivery device preferably comprises an electric motor adapted to move a movable output member, e.g. a motor shaft, plunger rod or movable element of the reservoir, in a dispensing direction for dispensing the liquid drug from the reservoir. The method according to the invention comprises the steps of
a) Gradually increasing or ramping-up a motor drive current for a motor (33) of the drug delivery device (1) from a zero value or a motor current that is lower than a motor current required to move a moveable output member by driving the motor for dispensing the drug;
b) Continuously measuring, by a position sensor or motion sensor, a position or movement of the output member (32) which is movable by the motor (33);
c) Determining a minimal motor drive current required to initially move or displace the output member (32) from standstill or halt;
d) Concluding, from an evolution (progression or curve) of successively (contiguously) determined minimal motor drive current values, a delivery device operation status.

In order to obtain a plurality of determined minimal motor drive current values the above steps a) to c) are repeated.

The method allows a monitoring of an evolution, a progression or curve of the minimal motor drive current in function of time or in function of position (and thus in function of a dispensed volume). The minimal drive motor current is the current required to start working the motor, namely, to initially move the output member from a non-moving steady state to a moving or drive state. This minimal motor drive current value may vary over time during use of the drug delivery device. Reasons for different values are, for example, the friction within the device, a biased or released powertrain within the device, different fill levels of the drug reservoir and degradation of material properties. However, under normal working conditions the minimal motor drive current values do not exceed a limited range. In case of a malfunction or system fault (as described in detail below) the minimal motor drive current required to drive the motor increases significantly compared to the minimal motor drive current values in an unobstructed condition.

Usually, automatic drug delivery devices with an electric motor are driven in an interval mode. That means the motor is driven for several short burst moving the plunger rod in a dispensing direction in short moving intervals. The motor is thus driven in a stop-and-go mode. Accordingly, several intervals or bursts may occur during dispensing of a single dose.

According to the invention in step a.) to c.) for every previously set dose, driving interval or burst the minimal motor drive current and preferably the corresponding time or position value are determined and recorded and preferably stored in an electronic module or memory in the drug delivery device. That means for a plurality of dispensed doses or driving intervals a sequence of contiguous minimal motor drive current values in function of time or in function of position (or dispensed volume) can be determined (evolution or progression of the recorded minimal drive current values).

The minimal motor drive current values are representative of the function of the delivery device. As the motor drive current is proportional to a motor force the minimal motor drive current values represent, for example, an increase or decrease of a motor force required to move the output member. Hence, a change of the minimal motor drive current and thus a change of the motor force can represent a malfunction of the delivery device (a change of the operational state). For example, if occlusion is present in the fluid path, if the drivetrain is blocked or a dispensing movement is in any other way hindered a higher minimal motor drive current is required to move the output member or the output member does not move at all.

If, for example, more than one absolute value of the minimal motor drive current values exceeds a predefined threshold a notification or alarm can be issued. In an other embodiment a rate of change of the determined minimal motor drive current values can be calculated and monitored in order to detect a malfunction of the device.

In contrast to prior art approaches the method according to the present invention allows a very earlier and reliable detection of a malfunction or a system fault. Namely, in contrast to known monitoring systems the motor will not only stop when a maximal drive current (maximal motor torque) is applied or if a predefined threshold is reached. The monitoring according to the invention allows to detect a critical minimal motor drive current (and thus a critical motor force) at a very early stage. In other words, as soon as the minimal motor drive current changes in a specific manner a malfunction can be reliably detected well before increasing the motor drive current to a maximal current value.

The drug delivery device may be an electronic injection device pen including an electric motor for moving a plunger rod to dispense the drug. For example, it may be a reusable electronic injection pen or it may be a patch injector applicable onto the skin of the user for the duration of the injection. Alternatively, the drug delivery device may be an infusion system, for example, a conventional medical drug pump such as an insulin pump with tubing or it may be a drug patch pump without tubing that is attachable directly onto the skin of the user, both comprise a driving unit including an electric motor.

The term "injection system" or "injector" refers to a device that is removed from the injection site after each medication event or drug delivery process, whereas the term "infusion system" refers to a device with a cannula or needle that remains in the skin of the patient for a prolonged period of time, for example, several hours.

The drug delivery device preferably comprises an electronic module or electronic unit including processing means or a controller, for example, a microcontroller or FPGA for carry out the above mentioned steps. The electronic module preferably comprises a data storage unit for storing the captured data and/or sensor signal data and the determined or calculated data. Furthermore, the drug delivery device preferably includes a communication unit configured to transmit data between the electronic unit and an external device such as a server, user mobile device or a computer of a healthcare facility.

The operational status is the working condition or status of the dispensing mechanism of the delivery device. Thus, if, for example, the output member does not move anymore due to an obstruction of if a drive force of the motor is required to move the output member increases in an unexpected manner the operational status changes from a normal working state to an error state.

The electric motor in form of a rotational or linear motor of the device is driven by the motor drive current, preferably controlled by a controller. Usually a voltage is supplied and controlled and the motor drive current applies according to the supplied voltage. The delivery device includes the movable output member which is set in rotation or in linear movement by the motor. The output member in turn is adapted to dispense the liquid drug out of the reservoir. Examples of the output member are a motor shaft, a plunger rod, a drive sleeve, a plunger inside the reservoir or any drive member of a dispensing mechanism of the delivery device.

According to the invention the motor drive current starts from a zero value or low value such that the motor does not yet work. Subsequently, the motor drive current is gradually or continuously increased. Thus, the controller ramps up the motor drive current until the motor starts working and the motor output member starts to move (rotation or linear movement depending on motor type). An instantaneous motor drive current when the motor starts to move the output member from a standstill is named herein as minimal motor drive current. That minimal motor drive current value is captured and stored in the electronic module every time the current is ramped up (for each dispensed dose, driving interval or burst).

In the present context, the terms "substance", "drug", "medicament" and "medication" are to be understood to include any flowable medical formulation suitable for controlled administration through a means such as, for example, a cannula or a hollow needle, and comprises a liquid, a solution, a gel or a fine suspension containing one or more medical active ingredients. A medicament can be a composition comprising a single active ingredient or a pre-mixed or co-formulated composition with more than one active ingredient present in a single container. Medication includes drugs such as peptides (e.g., insulin, insulin-containing drugs, GLP-1 containing drugs or derived or analogous preparations), proteins and hormones, active ingredients derived from, or harvested by, biological sources, active ingredients based on hormones or genes, nutritional formulations, enzymes and other substances in both solid (suspended) or liquid form but also polysaccharides, vaccines, DNA, RNA, oligonucleotides, antibodies or parts of antibodies but also appropriate basic, auxiliary and carrier substances

The term "distal" is meant to refer to the direction or the end of the drug delivery device carrying an injection needle or an injection cannula, whereas the term "proximal" is meant to refer to the opposite direction or end pointing away from the needle or cannula.

In a preferred embodiment the method further comprises the steps of determining a rate of change of a minimal drive current [MOJ-YA1] from the evolution of the successively determined minimal motor drive current values (rate of change over a plurality of recorded minimal drive current values) to determine or conclude the operational state, namely a malfunction.

That means based on stored minimal motor drive current values and the time or position values a rate of change can be calculated, namely the derivative of the minimal drive current curve. The determination or calculation is preferably carried out by a controller of the drug delivery device.

A specific rate of change of the captured minimal drive current values may be indicative of a malfunction of the device. The monitoring of the rate of change allows to even earlier detect a malfunction. Preferably, the absolute values of the minimal motor drive current values are monitored too to reliably detect a change in the operational state of the device.

The method preferbly further includes the step of comparing if the determined rate of change of minimal drive current values exceeds a predefined threshold (of a rate of change).

Consequently, it can be determined whether the rate of change lies below, above a threshold or within a predefined threshold band or range. In case the determined rate of change exceeds the threshold, e.g. is above, below or outside a band of the threshold, the controller may send, for example, a message or an alert to the user or a further action may be triggered.

A sequence of sucessively determined minimal motor drive current values is preferably analyzed by the controller during a dispensing period. That means a progression or curve of the plurality of minimal drive current values is analyzed. That allows to efficiently compare the determined minimal motor drive current values and also a progression of the values for a period of time or for a travel of the movable output member, e. g. during a dispensing process. Hence, a continuous monitoring of the minimal motor drive current during the dispensing process is enabled.

As soon as a minimal motor drive current exceeds a threshold for a particular period of time (within a time frame) or for a particular travel it can be concluded that the delivery device does not work properly. Periodically analyzing the captured minimal motor drive current values within a predefined period of time or within a predefined travel length or travel path allows to detect reliably a malfunction or a system fault on an early stage during operation of the motor.

The sequence of minimal motor drive current values in form a signal is preferably processed by an analog or digital filter. The filtered signal has a smooth progression and noise can be effectively removed by the filter. Hence, filtering the signal allows a reliable analysis and an easier further processing of the signal.

The filter used is preferably a digital finite impulse response filter (FIR filter). The FIR filter has the advantage that it is inherently stable as the output of the FIR filter is a sum of a finite number of finite multiples of filter input values. In contrast to other filter types the FIR filter does only use input values and does not use previous samples of an output signal.

Preferably, the minimal motor drive current determined according to above mentioned steps a) to c) is exclusively recorded at the beginning or on the first time before dispensing a previously set dose. Hence, every time the controller gradually increases the motor current in order to start dispensing a dose the minimal motor drive current is recorded. However, the minimal drive current is preferably only recorded at the first ramp-up step and for subsequent ramp-up steps (several driving intervals or bursts during dispensing of a dose) the minimal motor drive current values are not recorded as these subsequent values may be influenced by transient phases because the powertrain may be biased after several driving intervals or bursts.

Alternatively, also the minimal drive current values of subsequent ramp-up steps are recorded and may be used for a secondary control threshold. That means the recorded minimal motor drive current values of subsequent ramp-up steps may be compared to a second and different (e.g. higher) threshold than the above mentioned first threshold. In this way additional conditions during dispensing may be monitored, for example, a behavior or progression of forces within the drivetrain of the dispensing mechanism.

As commonly known the motor torque is proportional to the winding current in the motor. Hence an instantaneous drive force or motor torque can be periodically determined and recorded based on an instantaneous drive current. As every minimal motor drive current value is recorded a progression of the motor torque values in function of time or in function of travel path can be determined. Therefore, the method according to the invention allows to precisely determine the motor torque without a separate, costly and bulky force sensor.

A progression of the drive current in function of time or in function of travel has preferably the shape of a ramp. That means the motor drive current is gradually and linearly increased from a start zero value or start current value to the captured minimal motor drive current. In this way the minimal motor drive current can be determined in an efficient way.

Alternatively, the motor drive current is non-linearly or exponentially increased.

Once the minimal motor drive current is determined the controller preferably switches from a ramp up mode to a normal drive mode, meaning that the motor is driven with a predefined or normal drive current to move the motor output member with a constant speed. The motor is preferably a stepper motor. In this case the normal drive mode includes a driving of the motor in a full step, half step or micro stepping mode preferably without using any sensor or feedback signal. Thus, the stepper motor is driven in the usual stepping mode after the above described ramp-up procedure.

Preferably, the electric motor is a rotational motor and the output member is a rotational motor shaft. The movement and position of the motor shaft is easy to measure, for example, with a motion sensor, position sensor or accelerometer.

Preferably, the drug delivery device includes a motor encoder adapted to measure a movement of the motor output member. The encoder signal can used during the period when the motor drive current is gradually increased to detect an initial motion of the motor output member. Depending on the motor type a linear or rotational encoder can be used.

Alternatively, another position or motion sensor in the drug delivery device adapted to measure a motion in the drivetrain may be used.

The drug delivery device preferably comprises a computer readable medium for storing a computer program comprising instructions which, when executed by a computing device of the drug delivery device, cause the computing device to carry out the above described method. The computing device may be a controller, a FPGA, a microprocessor or a control circuit in the electronic module of the drug delivery device.

The drug delivery device comprises preferably a plunger rod for dispensing a liquid drug from a reservoir, the motor for moving the plunger rod, a computing device and the computer-readable medium.

In a preferred embodiment the drug delivery device is an injection device, in particular an injection pen. The injection device preferably comprises a reservoir unit or reservoir holder including the reservoir with liquid drug and a drive unit including the motor.

The injection device may be a disposable, reusable or semi-disposable device. The latter usually comprises a disposable reservoir unit that can be replaced and discarded after an injection or if the reservoir is empty. Subsequently, a new reservoir unit may be attached to a reusable drive unit in order to prepare the device for a new injection.

Alternatively, as mentioned above the drug delivery device may be an infusion device such as a conventional drug pump or a patch pump.

### BRIEF DESCRIPTION OF THE DRAWINGS

The subject matter of the invention will be explained in more detail in the following text with reference to preferred exemplary embodiments which are illustrated in the attached drawings, in which:
- Fig.1: depicts a side view of a drug delivery device in form of an electronic injection pen according to first embodiment of the present invention;
- Fig.2: depicts a sectional view of the injection pen of figure 1;
- Fig.3: depicts a perspective view of a drug delivery device in form of a patch injector according to a second embodiment;
- Fig. 4: depicts a sectional view of the patch injector of figure 3;
- Fig. 5: depicts a graph of a pull-in torque of a prior art delivery device;
- Fig. 6: depicts a graph of a pull-in torque with a filtered signal according to the present invention.

The reference symbols used in the drawings, and their primary meanings, are listed in summary form in the list of designations. In principle, identical parts are provided with the same reference symbols in the figures.

### DETAILED DESCRIPTION OF PREFERRED EMBODIMENTS

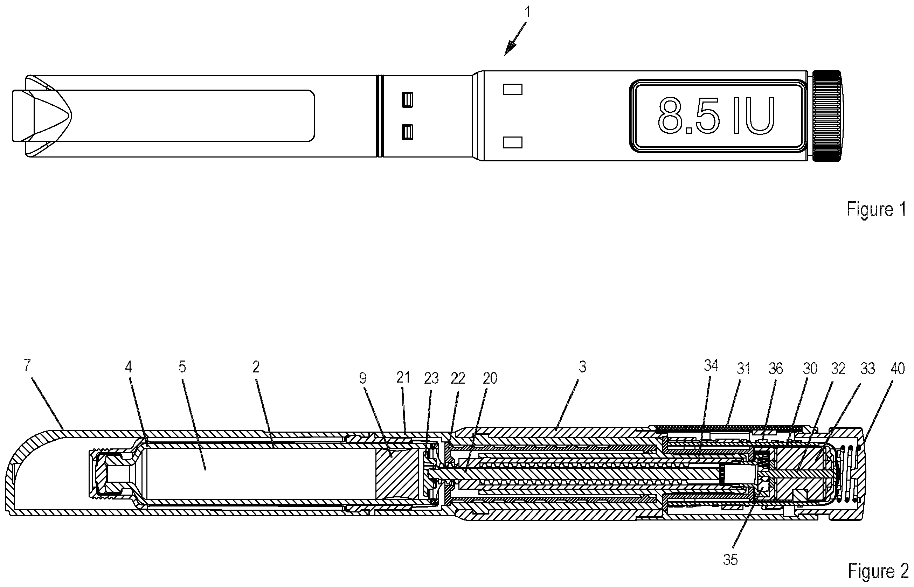

In the present description the term "distal" refers to the side where the needle is attached. This is on the left hand side in the figures 1 and 2. The term "proximal" refers to the opposite side and is on the right hand side in figures 1 and 2.

Figures 1 and 2 show a drug delivery device according to a first embodiment of the invention. The delivery device is a semi-disposable electronic injection pen 1. The pen 1 includes a disposable assembly 2 and a reusable assembly 3, shown in Figure 2.

Figure 2 depicts a sectional view of the injection pen 1 of figure 1. The disposable assembly 2 is formed by a cartridge unit 2 (or reservoir unit) including a reservoir holder or cartridge holder 4 and a reservoir in form of a cartridge 5 containing liquid drug. The cartridge unit 2 is releasably attachable to the reusable assembly 3.

The reusable assembly 3 comprises an automatic drive unit 30 and a display 31. The automatic drive unit 30 includes an electric motor 33 in form of a stepper motor, for moving a plunger rod 20, a sleeve-shaped automatic drive member 34, a gear 35 connecting a motor shaft 32 of the motor 33 to the automatic drive member 34, a printed circuit board (PCB) 36 with a controller (in form of a microcontroller or FPGA, not shown) for controlling the electric motor 33, an energy source in form of a rechargeable battery, a data storage module, a communication module and an encoder (all not shown) for sensing the movement of the motor shaft 32 and thus the movement of the plunger rod 20. The reusable assembly further includes a spring and a dose knob 40.

The cartridge unit 2 includes an interface housing 21, the cartridge holder 4, the cartridge 5, a mechanics holder 22 and the plunger rod 20 with a flange 23 pivotally mounted on a distal end of the plunger rod 20. A pen cap 7 can be mounted on a needle assembly (not shown) attachable to a distal end of the cartridge holder 4.

If the motor 33 of the injection pen 1 is activated, for example, via dose knob by the user, the motor shaft 32 rotates and the rotational movement is transferred via gear 35 to the automatic drive member 34. The latter in turn is splined to the plunger rod 20 and thus rotates the plunger rod 20. As the plunger rod 20 is screwed into the mechanics holder 22 of the injection pen 1 and is moved in distal dispensing direction. Therefore, a piston 9 inside the cartridge 5 is moved in dispensing direction which causes the liquid drug to dispense out of the cartridge 5.

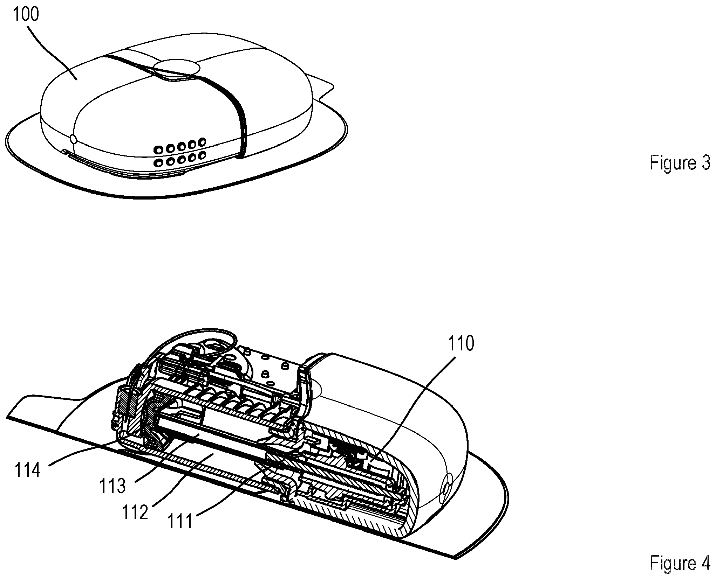

Figures 3 and 4 show a further embodiment of the present invention. The drug delivery device in this second embodiment is a patch pump 100 adapted to be attached to the skin of the user for a prolonged time. Figure 4 depicts a sectional view wherein a part of the housing is not shown. The patch pump 100 includes a drive mechanism with an electric motor 110, a motor encoder and a controller (not shown). The motor rotates a piston 111, which in turn moves a hollow plunger rod 113 with a flange 114 in a dispensing direction to dispense the liquid drug from a reservoir 112.

The method according to the present invention can be applied to the shown delivery devices according to the first and second embodiment in figure 1 to 4 but also to any other electronic delivery device including an electric motor such as, for example, a conventional drug pump, a patch injector or a fully reusable injection pen.

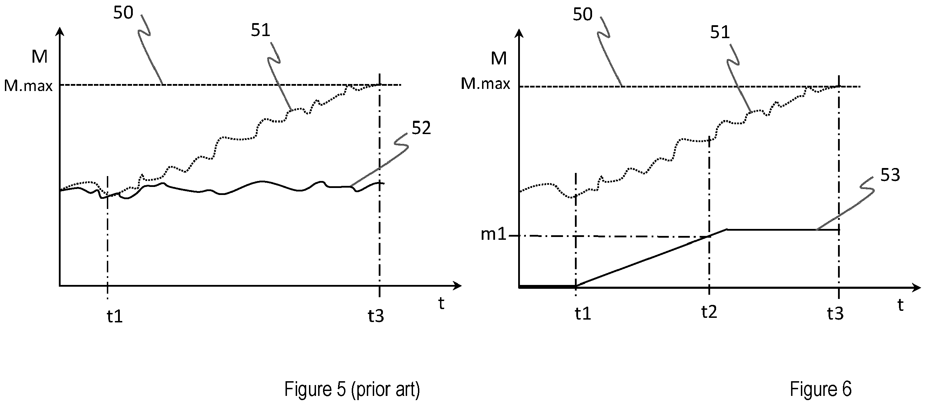

In the following the controlling and driving of the motor of the delivery device and the monitoring of a motor drive current (motor input current) and a corresponding motor pull-in torque is descripted with respect to figure 5 and 6.

Figure 5 depicts a graph representing captured pull-in torques of a stepper motor in function of time. Hence, the horizontal axis of the graph represents the time line whereas the vertical axis of the graph represents the pull-in motor torque of the motor.

Line 50 in the graph marks a maximal possible pull-in torque of the motor with a maximum motor drive current. Line 52 depicts a plurality of pull-in torques recorded during normal use during normal and unobstructed working condition. The dotted line 51 represents recorded pull-in torques in case of a system fault. Namely, from time point t1 an occlusion occurs in the fluid path or dispensing of the fluid from the reservoir is in any other way affected in that a higher motor torque is required to move the plunger rod or drive member.

According to prior art approaches (figure 5) the controller receives a signal from a force sensor and increases the drive current accordingly in order to increase the motor torque. In this way, the drive current rises until a maximum motor current or threshold is reached and hence the maximum pull-in torque as depicted with line 50 is reached. At the maximum "m.max" or threshold the controller stops the motor and issues a notification or alarm to the user. Hence, prior art approaches usually detect a system fault by means of a force sensor in the delivery device.

In the following the driving of the motor and monitoring of the drive current and pull-in torque according to the present invention is descripted with respect to figure 6. Figure 6 shows the same curve of pull-in torques as in figure 5 but additionally the graph includes a curve 53 representing a filtered signal of the captured minimal drive current values as descripted bellow in detail.

To start a dispensing process the controller starts to apply a voltage and a corresponding current is provided to the motor. This starting current is lower than a drive current required to initially move the motor shaft. The input drive is monitored by the controller or is periodically measured, for example, by means of a shunt resistor. The rotation and position of the motor shaft is measured by the motor encoder having a resolution configured to detect small movements. In particular, the motor encoder is adapted to detect the smallest possible motor step.

The controller gradually and linearly increases the drive current. Hence, the current provided to the motor is ramped-up. As soon as the encoder detects a rotational movement of the motor shaft the controller records the actual or instantaneous measured drive current which is named here as minimal drive current. The controller then switches from a ramp up mode to a normal stepper drive mode, e.g. a full step, half step or a micro stepping mode for driving the motor a predefined driving interval or burst.

The described determination of the minimal drive current is repeated every time at the beginning of a dispensing of a dose. Alternatively, the procedure is repeated several times during dispensing of a dose (e.g. for every burst during a dispensing process). The captured and recorded minimal drive current values are stored in the storage unit of the injection pen. A sequence of contiguous minimal drive current values are filtered by a FIR filter in order to reduce noise and to build a smooth progression of the data signal.

The filtered signal is depicted as curve 53 in figure 6. The curve 53 is smooth and has a reduced noise. Based on the filtered and processed signal the controller periodically calculates a rate of change of the minimal drive current values (calculation of derivation). The controller compares the calculated rate of change with a predefined threshold of rate of change. The threshold is chosen in a manner that an exceeding of the threshold rate of change is reliably indicative of a malfunction or a system fault. That means if the minimal drive current values and the corresponding pull-in moment (which is proportional to the input current as known) increase in a particular manner a malfunction such as occlusion is reliably detected.

As depicted in figure 6 after time point t1 the determined minimal drive current increases after an occlusion (or any other malfunction) occurred for the first time at time point t1. Namely, if in a given time frame or travel path the threshold of the rate of change is exceeded the controller is able to detect the malfunction at time t2 and at the level of "m1". That is well before time t3 when prior art approaches are able to detect the fault (reaching maximum value "m.max").

In case the calculated rate of change of minimal drive current values exceeds the threshold the controller stops driving the motor and sends a notification to the display of the delivery device or via communication unit to an external user device. Additional steps can be initiated depending on the configuration.

Besides the monitoring of the rate of change the controller monitores the absolute values of the determined minimal drive current in a second control loop. In case the absolute values exceed a threshold within a defined period the controller sends a notification too.

In addition to the calculation of the rate of change the controller periodically determines the pull-in torque based on the minimal drive current and records and stores the pull-in torque values. Information about an actual pull-in torque is indicative of the current working condition of the injection system. Therefore, by monitoring the minimal drive current values an instantaneous force can be determined without a force sensor.

While the invention has been described in detail in the drawings and foregoing description, such description is to be considered illustrative or exemplary and not restrictive. Variations to the disclosed embodiments can be understood and effected by those skilled in the art and practising the claimed invention, from a study of the drawings, the disclosure, and the appended claims. In the claims, the word "comprising" does not exclude other elements or steps, and the indefinite article "a" or "an" does not exclude a plurality. The mere fact that certain elements or steps are recited in distinct claims does not indicate that a combination of these elements or steps cannot be used to advantage, specifically, in addition to the actual claim dependency, any further meaningful claim combination shall be considered disclosed.

### LIST OF DESIGNATIONS

- 1: injection pen
- 2: disposable assembly
- 3: reusable assembly
- 4: cartridge holder
- 5: cartridge
- 7: cap
- 9: piston

- 20: plunger rod
- 21: interface housing
- 22: mechanics holder
- 23: flange

- 30: automatic drive unit
- 31: display
- 32: motor shaft
- 33: electric motor
- 34: automatic drive member
- 35: gear
- 36: PCB
- 40: dose knob

- 100: patch pump
- 110: motor
- 111: plunger
- 112: reservoir
- 113: piston rod
- 114: flange

## Claims

1. Method for monitoring a dispensing process of a liquid drug with a drug delivery device (1), the method comprising the steps of
a) Gradually increasing a motor drive current for a motor (33) of the drug delivery device (1);
b) Measuring, by a sensor, a position or movement of an output member (32) movable by the motor (33);
c) Determining a minimal motor drive current required to initially move the output member (32) from standstill;
d) Concluding, from an evolution of successively determined minimal motor drive current values, a delivery device operation status.

2. Method according to claim 1, comprising the step of determining a rate of change from the evolution of the successively determined minimal motor drive current values to conclude the delivery device operation status.

3. Method according to claim 2, comprising the step of comparing if the rate of change exceeds a predefined threshold.

4. Method according to any of claims 1 to 3, wherein a sequence of sucessively determined minimal motor drive current values is analyzed during a dispensing period.

5. Method according to claim 4, wherein the sequence is filtered.

6. Method according to claim 5, wherein the filter is a finite impulse response filter.

7. Method according to any of claims 1 to 6, wherein the minimal motor drive current determined according to steps a) to c) is recorded exclusively at the beginning of a dispensing of a previously set dose.

8. Method according to any of claims 1 to 7, wherein a drive force of the motor (33) is periodically determined and recorded based on the determined minimal motor drive current.

9. Method according to any of claims 1 to 8, wherein the motor drive current is linearly increased until the output member (32) starts to move.

10. Method according to any of claims 1 to 9, wherein the motor (33) is a stepper motor and wherein the motor is driven in a stepping mode preferably without a sensor signal after the minimal motor drive current is determined.

11. Method according to any of claims 1 to 10, wherein the output member (32) is a motor shaft.

12. Method according to any of claims 1 to 11, wherein the position or movement is measured by a motor encoder.

13. Computer program comprising instructions which, when executed by a computing device, cause the computing device to carry out the method according to any of claim 1 to 12.

14. Drug delivery device (1) comprising a plunger rod (20) for dispensing a liquid drug from a reservoir (5), the motor (33) for moving the plunger rod (20) and a computer-readable medium where the computer program of claim 13 is stored.

15. Drug delivery device (1) according to claim 14, wherein the device is an injection device.
